# EUROPEAN PATENT APPLICATION

(11) **EP 4 431 093 A1**
(43) Date of publication of application: **18.09.2024**
(21) Application number: 22889347.5
(22) Date of filing: 03.11.2022
(51) Int. Cl.: A61K 31/4152, A61K 31/045, A61P 25/28

(54) **APPLICATION OF COMPOSITION CONTAINING EDARAVONE AND DEXBORNEOL IN IMPROVING OR TREATING COGNITIVE IMPAIRMENT**

(30) Priority: 08.11.2021 CN 202111311631
(71) Applicant: Neurodawn Pharmaceutical Co., Ltd., Jiangsu 211199 (CN); Simcere Pharmaceutical Co., Ltd., Nanjing, Jiangsu 210032 (CN)
(72) Inventor: ZHANG, Zhengping, Nanjing, Jiangsu 210046 (CN); WANG, Lei, Nanjing, Jiangsu 210046 (CN); CHEN, Rong, Nanjing, Jiangsu 210046 (CN); YANG, Shibao, Nanjing, Jiangsu 210046 (CN); REN, Jinsheng, Nanjing, Jiangsu 210046 (CN)
(74) Representative: Lavoix
(86) International application number: PCT/CN2022/129456
(87) International publication number: WO 2023/078325

(57) **Abstract**

An application of a composition in improving or treating cognitive impairment. The composition contains 3-methyl-1-phenyl-2-pyrazolin-5-one or a pharmaceutically acceptable salt thereof and borneol or dexborneol. The cognitive impairment comprises Alzheimer's disease (AD), vascular dementia (VD), mild cognitive impairment (MCI), and other types of dementia.

## Description

The present application claims the right of priority for Chinese patent application no. 202111311631.1, filed with the China National Intellectual Property Administration on November 08, 2021 and entitled "APPLICATION OF COMPOSITION CONTAINING EDARAVONE AND DEXBORNEOL IN IMPROVING OR TREATING COGNITIVE IMPAIRMENT", which is incorporated herein by reference in its entirety.

### Technical Field

The present invention belongs to the pharmaceutical field and relates to an application of a composition of 3-methyl-1-phenyl-2-pyrazolin-5-one or a pharmaceutically acceptable salt thereof and borneol in treating cognitive impairment.

### Background

Dementia is a syndrome that is centered on acquired cognitive impairment and leads to an obvious decline in the activities of daily living, ability to learn, work capacity, and ability of social communication of patients. The cognitive impairment of patients involves memory, learning, orientation, understanding, judgment, calculation, language, visual-spatial function, and analysis and problem-solving ability, and is often accompanied by mental, behavioral, and personality disorders at a certain stage during the course of the disease. Dementia includes degenerative and non-degenerative dementia, the degenerative dementia including Alzheimer's disease (AD), dementia with Lewy bodies (DLB), frontotemporal lobe degeneration (FTLD), etc.; the non-degenerative dementia including vascular dementia (VaD), normal pressure hydrocephalus, and dementia caused by other diseases such as craniocerebral injury, infection, immune diseases, tumors, poisoning and metabolic diseases (Guidelines for the diagnosis and treatment of dementia and cognitive impairment in China (2018), National Medical Journal of China, 2018.98 (13), p: 965-970).

AD is a neurodegenerative disease that starts slowly and gradually worsens over time, and is currently the only disease for which there is no cure, prevention or even delay of progression. AD ranked seventh among the top ten causes of death in the world announced by WHO in 2020. The most common early symptom of AD is short-term memory loss (difficulty in remembering recent events). As the disease progresses, symptoms such as language disorder, disorientation (including easily getting lost), emotional instability, loss of motivation, inability to self-care, and behavioral issues may gradually emerge. As the disease worsens, patients often withdraw from family and society. Gradually, bodily functions are lost, ultimately leading to death. Although the course of the disease varies from person to person, the average life expectancy after diagnosis is about 3 to 9 years.

According to World Alzheimer Report 2021 released by the Alzheimer's Disease International, as of 2019, there was a total of 55 million Alzheimer's patients worldwide. With the trend of population aging, the estimated number of AD patients worldwide will reach 78 million by 2030 and 129 million by 2050. China has entered the stage of rapid growth of aging, and the number of elderly people continues to climb. By the end of 2018, the number of elderly people aged 60 years and older in China reached 249 million, accounting for 17.9% of the total population; and the number of elderly people aged 65 years and older in China reached 166 million, accounting for 11.9% of the total population. According to the national epidemiological survey on mental diseases in China in 2017, the prevalence of senile dementia among elderly people aged 65 years and older in China was 5.56% (The Lancet Psychiatry, 2019. 6(3): p. 211-224). Meta-analysis in recent years has shown that the overall prevalence of dementia in people aged 60 years and older in China was 5.3%. The number of patients with dementia aged 60 years and older in China is estimated to be 10-11 million, 60% of which are patients with AD (The Lancet Neurology, 2020. 19(1): p. 81-92.).

Five "old drugs" have been approved by the FDA to relieve certain symptoms in AD patients, including four cholinesterase inhibitors tacrine (exited the market due to hepatotoxicity), donepezil, galantamine and rivastigmine, and one NMDAR antagonist memantine (Molecules, 2020. 25(24)). In addition, Ganlutena Jiaonang ( 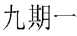 ^{®}, GV-971, low molecular acidic oligosaccharide) targeting gut-brain axis, which was conditionally approved by the NMPA in China in November 2019, improves the cognitive function of patients with mild to moderate AD (First Approval. Drugs, 2020. 80(4): p. 441-444). Aducanumab (Aduhelm^{®}, anti-Aβ monoclonal antibody), which was conditionally approved by the FDA on June 7, 2021, is an IgG1 monoclonal antibody drug targeting soluble and insoluble Aβ amyloid proteins, and is used for treating patients with mild cognitive impairment or mild AD (First Approval. Drugs, 2021. 81(12): p. 1437-1443.). However, there is currently no cure for AD with all approved drugs, and there is an urgent clinical need for drugs for treating AD that are more effective and more able to delay disease progression.

3-methyl-1-phenyl-2-pyrazolin-5-one (edaravone) is a small molecule compound that is capable of scavenging free radicals. At the in vitro level (*in vitro*), edaravone is capable of inhibiting the aggregation of Aβ₁₋₄₂ monomer as Aβ fibrils and disaggregating Aβ fibrils in a concentration-dependent manner in a concentration range of 0.156 to 5 µM; edaravone is capable of reducing the neurotoxicity of Aβ₁₋₄₂ to SH-SY5Y and primary cortical neurons, increasing cell viability and maintaining neurite growth. In SY-SY5Y (SH-SY5Y-APP695) stably expressing APP695 protein, edaravone (0.3 to 3 µM) is capable of reducing BACE1 expression, cleavage of APP and Aβ production in a concentration-dependent manner, inhibiting GSK-3β activation and reducing tau phosphorylation (Proc Natl Acad Sci U S A, 2015. 112(16): p. 5225-30). In addition, edaravone is capable of reducing H₂O₂- and glutamate-induced death of primary hippocampal neurons, protecting neuronal synaptic growth, and reducing peroxidation and excitatory neurotoxicity (Neurotoxicology, 2021. 85: p. 68-78). At the in vivo level (*in vivo*), edaravone is capable of improving the cognitive function in multiple rat or mouse models of dementia, and reducing the oxidative indicators, neuroinflammation, Aβ plaque formation, tau phosphorylation, etc. within the brain. In the rat model of scopolamine-induced dementia, oral administration of edaravone at 15 mg/kg/day for 7 consecutive days can improve scopolamine-induced spatial learning and long-term memory impairments (International Journal of Pharmacology, 2013. 9(4): p. 271-276). In the rat model in which oligomerized Aβ₁₋₄₀ is injected into the hippocampal dentate gyrus, intracerebral injection of Aβ and simultaneous administration of edaravone (5 mg/kg) at the same site can significantly improve the spatial learning and memory of rats in water maze, reduce the level of 4-hydroxy-2-nonenal (4-HNE) adduct in the hippocampus, and restore the increased AChE and ChAT enzyme activities due to injection of Aβ (Neurological Sciences, 2015. 36(11): p. 2067-2072). In the sporadic AD (SAD) rat models of intracerebroventricular injection of STZ (ICV-STZ)-induced learning and memory impairments, after ICV-STZ modeling, intraperitoneal administration of edaravone at 9 mg/kg/day for 14 consecutive days can significantly improve the spatial learning and memory (tested by Morris water maze test) and non-spatial long-term memory (tested by step-down passive avoidance test) of the ICV-STZ model rats; and can significantly restore changes in the levels of various oxidative stress indicators in the hippocampus and cerebral cortex caused by STZ modeling, including malondialdehyde (MDA), 4-HNE adducts, ·OH, protein carbonyl (PC), H₂O₂, superoxide dismutase (SOD), reduced glutathione (GSH), glutathione peroxidase (GPx) (NeuroToxicology, 2013. 38: p. 136-145). In addition, oral administration of edaravone (1, 3 and 10 mg/kg/day) for 28 consecutive days can also improve the cognitive function of ICV-STZ rats in water maze test and passive avoidance test, and reduce STZ-triggered oxidative stress, cholinergic dysfunction and inflammation within the brain (Eur J Neurosci, 2017. 45(7): p. 987-997). In the mouse model of AlCl₃/D-galactose-induced cognitive impairment, intraperitoneal administration of edaravone for 15 consecutive days significantly improves the cognitive function as tested by water maze test and step-down test (Neurotoxicology, 2021. 85: p. 68-78). In addition, in the rat model of L-methionine-induced vascular dementia, intraperitoneal administration of edaravone at 6 mg/kg/day for 9 consecutive weeks can significantly improve the spatial learning and memory of the model rats in eight-arm water maze test (Naunyn Schmiedebergs Arch Pharmacol, 2020. 393(7): p. 1221-1228). In the APP/PS1 transgenic mouse model, both prophylactic administration (mice aged 3 to 9 months) and therapeutic administration (mice aged 9 to 12 months) of edaravone at 12.6 mg/kg (intraperitoneal injection, twice a week) can improve the deficits in the cognitive function in water maze test, and reduce the Aβ plaque deposition, loss of neurons and synapses, neuroinflammation, tau protein phosphorylation (pSer396, pSer262, pSer199 and pThr231), oxidative stress, etc. within the brain. Also based on the oral bioavailability (38%) of edaravone in mice, oral administration (administration with drinking water) at 33.2 mg/kg/day in mice (aged 3-12 months) can also reduce Aβ plaque deposition within the brain and improve the cognitive function of mice (Proc Natl Acad Sci U S A, 2015. 112(16): p. 5225-30). In addition, oral administration of edaravone (8, 24 and 72 mg/kg/day, administration with drinking water) in APP/PS aged mice (aged 14 to 17 months) significantly improves the cognitive function of aged transgenic mice (Drug Design, Development and Therapy, 2018. Volume 12: p. 2111-2128). In the tau P301L transgenic mouse model of frontotemporal dementia (FTD), oral administration (administration with drinking water) of edaravone at 24 mg/kg/day for 3 consecutive months in transgenic mice aged 9 to 10 months and aged 21 months can significantly improve the cognitive function of the animals in water maze test and novel object recognition test, improve the motor deficits of the animals, and reduce the production of 4-HNE and 3-NT adducts, tau phosphorylation and neuro inflammation in the brain tissue (DOI: https://doi.org/10.21203/rs.3.rs-306628/v1).

The chemical structural formula of edaravone is as follows:

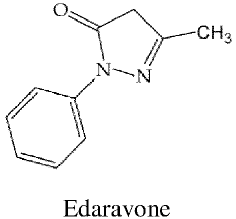

### (Molecular formula C₁₀H₁₀N₂O; molecular weight 174.20)

Dexborneol ((+)-borneol) is the main component (the 2020 edition of the Chinese Pharmacopoeia stipulates that the content of dexborneol in natural borneol is not less than 96%) of natural borneol, is a bicyclic monoterpenoid compound, and is present in the volatile oils of many Chinese herbal medicines. Dexborneol can regulate NF-κB and p38 signaling pathways, inhibit inflammatory neuro inflammation, and reduce multiple pro-inflammatory factors (TNF-α, IL-1β, etc.) and inflammatory proteins (iNOS and COX-2) within the brain (Eur J Pharmacol, 2014. 740: p. 522-31; Eur J Pharmacol, 2017. 811: p. 1-11). In addition, dexborneol can agonize inhibitory GABAAR receptors and regulate neuronal excitability injury (Front Pharmacol, 2021. 12: p. 606682).

The chemical structural formula of dexborneol is as follows:

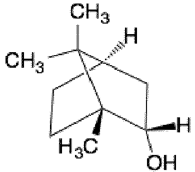

Dexborneol (Molecular formula Cl₁₀H₁₈O; molecular weight 154.25) Non-clinical animal experiments indicate that a combination of 3-methyl-1-phenyl-2-pyrazolin-5-one and dexborneol (mass ratio 4 : 1 to 1 : 1) can synergistically reduce cerebral infarct size (granted patent CN 101848711 B) and improve amyotrophic lateral sclerosis (ALS) (granted patent CN 107613976 B). In the research and development of new drugs for the treatment of stroke, the edaravone and dexborneol concentrated solution for injection (a combination of 3-methyl-1-phenyl-2-pyrazolin-5-one and dexborneol in a mass ratio of 4 : 1) has been approved by NMPA in 2020 for the treatment of ischemic stroke; the Y-2 sublingual tablet (a combination of 3-methyl-1-phenyl-2-pyrazolin-5-one and dexborneol in a mass ratio of 5 : 1) has undergone phase III study in China (Chinese Clinical Trial Registry No. CTR20210233) and phase I study in the United States (*ClinicalTrials.gov*NCT03495206)*.*

In light of the prior art, it is impossible to predict whether a combination of 3-methyl-1-phenyl-2-pyrazolin-5-one and dexborneol has a therapeutic effect on dementia and its related cognitive impairment, and it is also impossible to judge whether the existing combination of 3-methyl-1-phenyl-2-pyrazolin-5-one and dexborneol can produce a synergistic effect.

### Summary of the Invention

The objective of the present invention is to provide the use of a pharmaceutical composition in the preparation of a drug for improving or treating cognitive impairment, wherein the pharmaceutical composition contains 3-methyl-1-phenyl-2-pyrazolin-5-one (edaravone) or a pharmaceutically acceptable salt thereof and borneol.

Another objective of the present invention is to provide the use of a pharmaceutical composition in improving or treating cognitive impairment, wherein the pharmaceutical composition containing 3-methyl-1-phenyl-2-pyrazolin-5-one or a pharmaceutically acceptable salt thereof and borneol is administered to a patient in need thereof.

A further objective of the present invention is to provide a pharmaceutical composition, wherein the composition is used for treating cognitive impairment, and the composition contains 3-methyl-1-phenyl-2-pyrazolin-5-one or a pharmaceutically acceptable salt thereof and borneol.

A still another objective of the present invention is to provide the use of 3-methyl-1-phenyl-2-pyrazolin-5-one or a pharmaceutically acceptable salt thereof in combination with natural borneol in treating cognitive impairment.

Still further, the use of 3-methyl-1-phenyl-2-pyrazolin-5-one or a pharmaceutically acceptable salt thereof in combination with borneol in the pharmaceutical composition is capable of synergistically increasing the efficacy for the treatment of cognitive impairment.

In some embodiments of the present invention, the cognitive impairment is Alzheimer's disease (AD), vascular dementia (VD), mild cognitive impairment (MCI), and other types of dementia; preferably, the cognitive impairment is Alzheimer's disease, vascular dementia, and mild cognitive impairment.

In one embodiment of the present invention, the pharmaceutical composition is capable of improving the cognitive function in patients with Alzheimer's disease.

In another embodiment of the present invention, the pharmaceutical composition is capable of restoring the long-term potentiation (LTP) in the hippocampus and restoring the synapse function in the hippocampus of brain in patients with Alzheimer's disease.

In a still another embodiment of the present invention, the pharmaceutical composition is capable of improving the pathology of Alzheimer's disease, including amyloid plaques, astrogliosis, microgliosis, and tau protein hyperphosphorylation.

In the composition of the present invention, the weight ratio of the 3-methyl-1-phenyl-2-pyrazolin-5-one or the pharmaceutically acceptable salt thereof: borneol is 10 : 1 to 1 : 8; preferably 7.5 : 1 to 2.5 : 1; further preferably 5 : 1 or 4 : 1.

The borneol of the present invention is preferably a natural borneol, which is also known as dexborneol.

The pharmaceutical composition of the present invention may further comprise a pharmaceutically acceptable excipient.

An excipient is generally a pharmacologically inactive substance formulated with an active pharmaceutical ingredient ("API") for drug treatment. When dosage forms are produced, excipients are often used to increment preparations containing effective active ingredients (thus often referred to as "extenders", "fillers" or "diluents") to allow for convenient and accurate dispensing of bulk drugs. It may also serve different therapeutic enhancement purposes such as promoting drug absorption or dissolution or other pharmacodynamic considerations.

More specifically, the compositions of the present invention may be formulated into a pharmaceutical composition either alone or in combination with a pharmaceutically acceptable carrier or diluent, and may be formulated into a preparation in a solid, semi-solid, liquid or gaseous form, such as a tablet, a capsule, a powder, a granule, an ointment, a solution, a suppository, an injection, an inhalant, a gel, a microsphere and an aerosol. Thus, the administration of the compound can be achieved in a variety of ways, including oral administration, buccal administration, rectal administration, parenteral administration, intraperitoneal administration, intradermal administration, transdermal administration, intraductal administration, intra-articular administration, etc. The active agent may be systemic after administration, or may be located using regional administration or intramural administration, or using an implant that maintains the active dose at the implantation site.

The combination therapy used in the present invention may allow the dose in each monotherapy to be lower than those currently used in standard operations, while achieving significant efficacy, including efficacy exceeding the efficacy achieved when the any monotherapy is conventionally administrated. It should be readily understood by those skilled in the art that dose levels can vary with a particular compound, severity of symptoms, and susceptibility of a subject to side effects. Some particular compounds are more effective than others. The preferred dose of a specified compound can be readily determined by those skilled in the art by a variety of means. A preferred means is to measure the physiological efficacy of the specified compound. The use of combination therapy may allow the dose in each monotherapy to be lower than those currently used in standard operations, while achieving significant efficacy, including efficacy greater than the efficacy achieved when the any monotherapy is conventionally administrated.

### Brief Description of the Figures

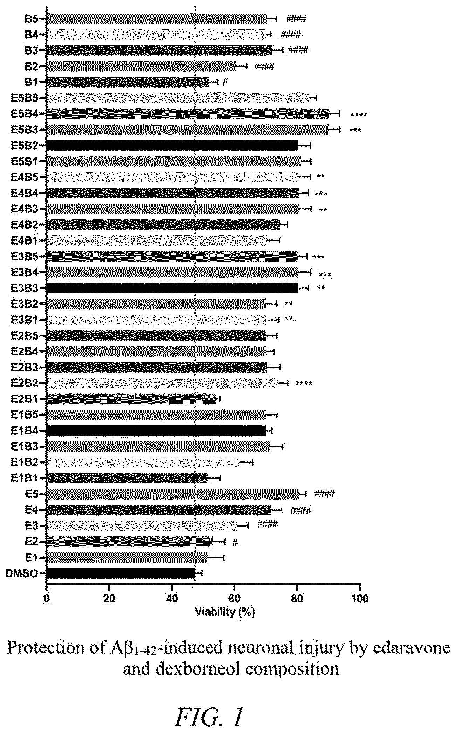

FIG. 1 Protection of Aβ₁₋₄₂-induced neuronal injury by edaravone and dexborneol composition;

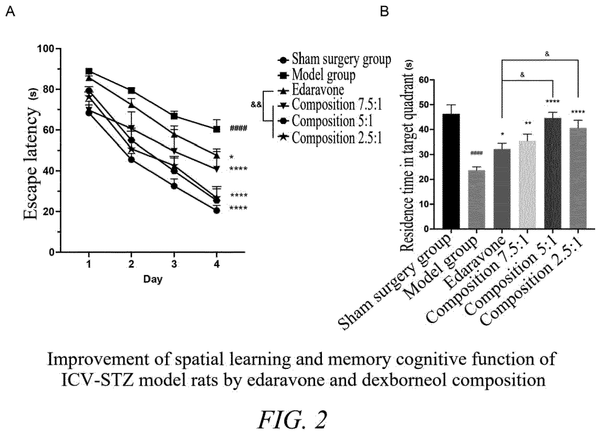

FIG. 2 Improvement of spatial learning and memory cognitive function of ICV-STZ model rats by edaravone and dexborneol composition;

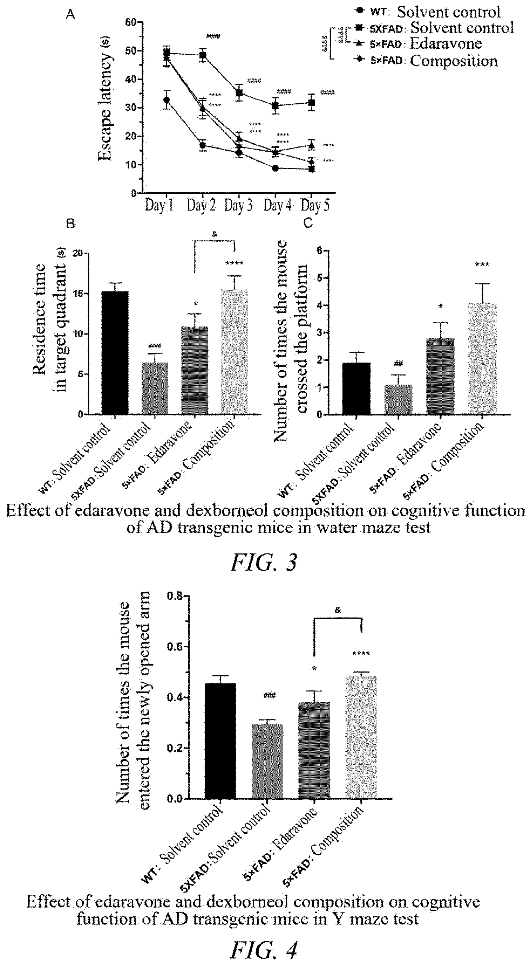

FIG. 3 Effect of edaravone and dexborneol composition on cognitive function of AD transgenic mice in water maze test;
FIG. 4 Effect of edaravone and dexborneol composition on cognitive function of AD transgenic mice in Y maze test;

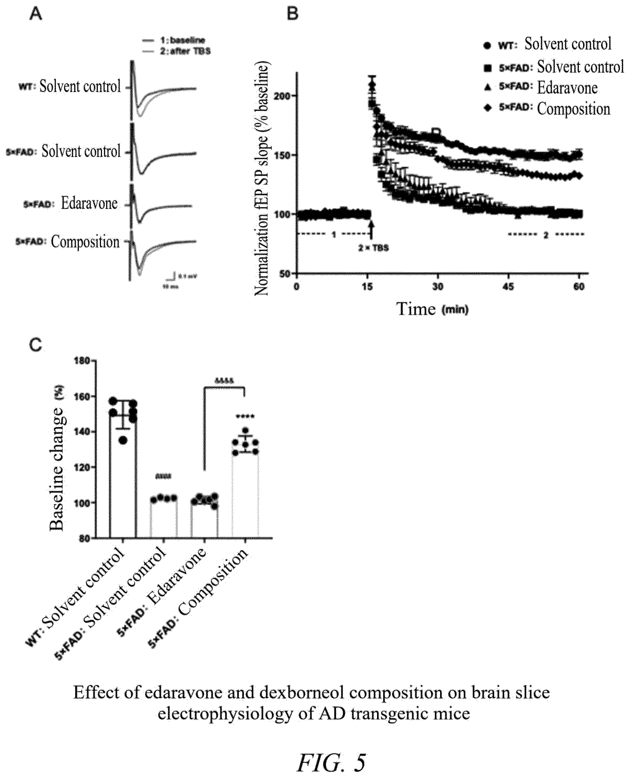

FIG. 5 Effect of edaravone and dexborneol composition on brain slice electrophysiology of AD transgenic mice;

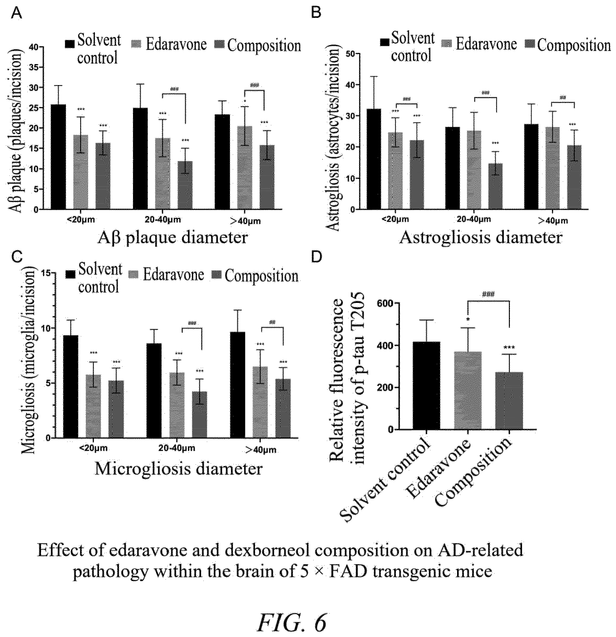

FIG. 6 Effect of edaravone and dexborneol composition on AD-related pathology within the brain of 5×FAD transgenic mice.

### Detailed Description of Embodiments

The following examples are intended to exemplify the present invention and should not be considered as limiting the present invention. The edaravone mentioned in the examples is 3-methyl-1-phenyl-2-pyrazolin-5-one.

### Example 1: Protection of Aβ₁₋₄₂-induced primary cortical neuronal injury by edaravone and dexborneol composition

### 1 Materials and methods

### 1.1 Animals

SD pregnant rats, Shanghai SLAC laboratory Animal Co., Ltd. (production license number: SCXK (Shanghai) 2017-0005)

### 1.2 Reagents and consumables

| **Name** | **Catalog No.Batch No.** | **Manufacturer** |
|---|---|---|
| Neurobasal | 21103-049 | Gibco |
| B27 | 17504-044 | Gibco |
| GlutaMax | 2110349 | Gibco |
| CellTiter-Glo | G7571 | Promega |
| Polylysine (PDL) | P6407 | Sigma |
| DMEM | 11995-040 | Gibco |
| Glucose-free DMEM | 1763966 | Gibco |
| Pennicillin Streptomycin (P/S) | 15140-122 | Gibco |
| Fetal bovine serum (FBS) | 10099-141 | Gibco |
| Cell Culture Plate | Corning 3610/96 well cell culture plate | Corning |
| Edaravone | / | Simcere Pharmaceutical Co., Ltd. |
| Dexborneol | KC20171205-1-2 | Jiangsu Simovay Pharmaceutical Co., Ltd. |
| CellCounting-Lite 2.0 | | Nanjing Vazyme |
| Luminescent Cell Viability Assay | LOT 7E322D9 | Medical Technology Co., Ltd. |

### 1.3 Preparation of primary cortical neurons

A SD pregnant rat at 18 days of pregnancy was sacrificed by cervical dislocation, the uteruses of the rat were dissected, the brain of E18 fetal rat was taken out, the cerebral cortical tissue of the fetal rat was isolated into an ice-cold DMEM, the meninge and blood vessels were removed from the cortical tissue under a dissecting microscope, the cortical tissue was transferred to an ice-cold DMEM and minced (about 1 mm³), and digested with trypsin at 37°C for 10 min, the digestion was terminated with FBS, a Pasteur pipette was used to perform gentle pipetting and dispersing, and screening was then performed using a 200-mesh sieve. The filtered cell suspension was transferred to a 15 mL centrifugal tube and centrifuged at 1000 rpm for 5 min. After the supernatant was discarded by sucking, the cell mass at the bottom of the centrifugal tube was gently pipetted and dispersed with a complete medium (Neurobasal + B27 + GlutaMax+1% P/S) preheated at 37°C. The cells were counted by a hemocytometer, diluted to 5 × 10⁵ cells/mL with a complete medium, and seeded in a 96-well plate coated with PDL (100 µ/well) for culture. Half of the medium was replaced with the complete medium the next day. The cells were cultured in vitro until day 11 when neurons differentiated and matured for oxygen and glucose deprivation test.

### 1.4 Aβ₁₋₄₂-induced primary cortical neuronal excitability injury test

The medium of the in vitro matured primary neurons was replaced with a complete medium containing different concentrations (1.2, 3.7, 11.1, 33.3, 100.0 µM) of edaravone (E), dexborneol (B) or an edaravone and dexborneol composition (EB), after incubation at 37°C for 30 min, oligomerized Aβ₁₋₄₂ (final concentration of 50 µM) was added, the culture was continued for 24 h, and the neuronal viability was tested. The cell viability of the primary neurons was tested using Luminescent Cell Viability Assay kit. The reagent was added at 100 µL/well according to the instructions, and shaken for 10 min, the chemiluminescence value (LUM) was read by a SpectraMax i3X (Molecule Device) multifunctional microplate reader, and the relative neuronal viability was calculated. Calculation formula: Relative neuronal viability V(%) = (LUM - LUM_{background})/(LUM_{normal control group} - LUM_{background group}) × 100%. LUM_{background} is the background reading of the cell-free medium well into which Luminescent Cell Viability Assay kit is added.

**Table 1 Orthogonal design of edaravone and dexborneol composition**

| DMSO | E1 | E2 | E3 | E4 | E5 |
|---|---|---|---|---|---|
| B1 | E1B1 | E2B1 | E3B1 | E4B1 | E5B1 |
| B2 | E1B2 | E2B2 | E3B2 | E4B2 | E5B2 |
| B3 | E1B3 | E2B3 | E3B3 | E4B3 | E5B3 |
| B4 | E1B4 | E2B4 | E3B4 | E4B4 | E5B4 |
| B5 | E1B5 | E2B5 | E3B5 | E4B5 | E5B5 |

| | | | | | |
|---|---|---|---|---|---|
| Notes: E stands for edaravone, B stands for dexborneol, ExBy stands for edaravone and dexborneol composition, x/y data 1-5 are concentrations of the compounds, 1.2, 3.7, 11.1, 33.3, and 100.0 µM, respectively. 5 replicate wells (n = 5) for drugs in each well were set. All compounds were firstly dissolved in DMSO, and the final concentration of DMSO in the medium was 0.2%. | | | | | |

### 1.5 Data statistics

Differences between two groups were analyzed using Uncorrected Fisher's LSD after One-way ANOVA was performed using Prism software (GraphPad). P < 0.05 indicated significant difference. #p < 0.05, ####p < 0.0001, comparison between Ex group or By group and DMSO group; **p < 0.01, ***p < 0.001, ****p < 0.0001, significant difference both between ExBy group and Ex group and between ExBy group and By group.

### 2 Experimental results

Oligomerized Aβ₁₋₄₂ leaded to a decrease in the cell viability of the primary neurons. Before Aβ₁₋₄₂ induction, pre-incubation and then administration of edaravone (3.7 to 100 µM) or dexborneol (1.2 to 100 µM) was capable of inhibiting Aβ-induced neurotoxicity in a concentration-dependent manner, increasing the cell viability. The orthogonal composition containing different concentrations (1.2, 3.7, 11.1, 33.3, and 100.0 µM) of edaravone and different concentrations (1.2, 3.7, 11.1, 33.3, and 100.0 µM) of dexborneol was capable of inhibiting Aβ-induced neuronal injury, especially the protective effects of the compositions E5B4 (3.3 : 1), E5B3(9 : 1), E4B5(1 : 3.3), E4B4(1 : 1), E4B3(3.3 : 1), E3B5(1 : 9), E3B4(1 : 3.3), E3B3(1 : 1), E3B2(3.3 : 1), E3B1(9 : 1) and E2B2 (1 : 1) were significantly superior to those of the corresponding individual components (Error! Reference source not found). Therefore, it was preliminarily determined that the composition of edaravone and dexborneol in the range of a molar ratio of 9 : 1 to 1 : 9 (a mass ratio of 10 : 1 to 1 : 8) had a synergistic effect.

### Example 2: Improvement of cognitive function of rats of intracerebroventricular STZ-induced dementia by edaravone and dexborneol composition

### 1 Experimental materials

### 1.1 Experimental animals

Wistar rats, male, body weight 250-300 g, 110 rats, clean-grade, provided by Shanghai SLAC laboratory Animal Co., Ltd., license number: SCXK (Shanghai) 2012-0002. Conventional feeding was performed before and after surgery, the room temperature was kept at 23°C-25°C, and the rats had free access to food and water.

### 1.2 Experiment instruments

Morris water maze (Topscan lite 2.0 analysis software, Clever Systems); Rat brain stereotaxic instrument (model 51600, Stoeling); Analytical balance (model AL104, Melttler Toledo).

### 1.3 Test drugs

Edaravone injection ( 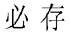 ^{®}), active ingredient 3-methyl-1-phenyl-2-pyrazolin-5-one. Edaravone and dexborneol compositions (weight ratios of 7.5 : 1, 5 : 1 and 2.5 : 1), which were ready-to-use, with a solvent control being 8% propylene glycol.

### 2 Experimental design

### 2.1. Experiment grouping and administration

The experimental animals were divided into 5 groups: sham surgery group, model group, edaravone group (6 mg/kg), edaravone and dexborneol (7.5 : 1) composition (edaravone 6 mg/kg, dexborneol 0.8 mg/kg), edaravone and dexborneol (5 : 1) composition (edaravone 6 mg/kg, dexborneol 1.2 mg/kg), and edaravone and dexborneol (2.5 : 1) composition (edaravone 6 mg/kg, dexborneol 2.4 mg/kg). The animals in the model group and sham surgery groups were given the corresponding volume of blank solvent (8% propylene glycol), all administrations were intraperitoneal injection administrations, and the administration volume is 10 mL/kg.

### 2.2 Experiment procedures

In addition to the sham surgery group, animals with successful surgery were randomly assigned to the model group and administration groups in order. According to the above dose setting principle, the animals in each group were given the corresponding drug intravenously 4 hours after the surgery, and then given the drug once daily for 14 consecutive days. Morris water maze test began on day 15. During the Morris water maze test, the animals were given the drug once daily before the test until the end of the experiment as a whole. The animals in each group were subjected to water maze test, and the test indicators were the escape latency in the hidden platform and the residence time in the original platform quadrant after withdrawal.

### 3 Experimental methods

### 3.1 Preparation of rat model of bilateral intracerebroventricular streptozotocin (STZ)-induced dementia

After anesthesia, the rat was fixed in a prone position on a stereotaxic instrument, a dorsal cervical median incision was performed to expose the skull, and taking the bregma as the coordinate, the lateral ventricular injection point was at 0.8 mm posterior to the bregma, and 1.5 mm lateral to the bregma, with a needle insertion depth of 3.6 mm. 10 µL of STZ (prepared with an artificial cerebrospinal fluid) was respectively injected into the bilateral ventricles using a microinjector within 1 min, and the needle was retained for 3 min. The skin was sutured after the injection. The first day dose was 1.5 mg/kg, the first day STZ injection method was repeated on the third day, and the total dose of STZ injection was 3 mg/kg. Equal volume of artificial cerebrospinal fluid was injected into the bilateral ventricles of the animals in the sham surgery group.

### 3.2 Morris water maze test

The Morris water maze pool was 160 cm in diameter, 50 cm in height, and 29 cm in water depth. The platform was 12 cm in diameter and 27 cm in height, and was fixed 2 cm below the water surface in the SE quadrant (target quadrant). A camera lens was placed above the center of the pool at a distance of 2 m from the bottom of the pool for synchronously recording the motion trajectories of rats. The water temperature was kept at 22 ± 1°C. The experiment lasted for a total of 5 days, including hidden platform trial and probe trial. The first 4 days were for the hidden platform trial, and the last 1 day was for the probe trial.

### (1) Hidden platform trial

This trial lasted for 4 days. The platform was fixed in the target quadrant (SE quadrant) during the trial, water entry was performed from the water entry points of the four quadrants, respectively, animals were gently placed in the water facing the sign on the pool wall during training, the time (escape latency) for the animal to find the platform was recorded, and then the animal was allowed to stay on the platform for 10 s. If the animal did not find the platform within 90 s, the latency was recorded as 90 s, and the animal was guided to stay on the platform for 10 s. After the training was completed, the rats were placed back to rearing cages and kept warm. Every day, the training was performed once at each of the 4 water entry points, and the mean of the 4 latencies was used as the performance for that day for statistical analysis. Every day, the order of water entry points for each animal was kept consistent, but the order of water entry for different animals in the same cage was different, so as to exclude the information communication among the animals in the group.

### (2) Probe trial

The platform was removed 24 h after the hidden platform trial was completed. Then water entry was performed from the water entry point opposite to the platform, the swimming path of rats within 120 s was recorded, and statistical analysis was performed on the residence time of the rats in the target quadrant. Animals were observed for spatial localization ability and change law during the probe.

### 3.3 Data statistics

Data were expressed as Mean ± SEM. Statistical analysis between groups was performed using Prism software (GraphPad, USA): One-way or Two-way ANOVA was used for comparison of two or more groups, and then Fisher's LSD test was selected for analysis. P < 0.05 indicated significant difference. ####p < 0.0001, comparison with sham surgery group; *p < 0.05, **p < 0.01, ***p < 0.001, ****p < 0.0001, comparison with model group; & p < 0.05, && p < 0.01, comparison between two groups indicated.

### 4 Experimental results

In the water maze hidden platform trial, compared with the sham surgery group, the intracerebroventricular injection of STZ (ICV-STZ) model had a significantly prolonged escape latency, indicating that the ICV-STZ rats had cognitive impairment. The intraperitoneal injection of edaravone (6 mg/kg) or edaravone and dexborneol (7.5 : 1, 5 : 1 and 2.5 : 1) compositions could significantly shorten the escape latency of the ICV-STZ rats. Compared with the edaravone administration group, the improvement in the learning function of the ICV-STZ model rats by the edaravone and dexborneol (7.5 : 1, 5 : 1 and 2.5 : 1) compositions was significantly superior to that by edaravone (Error! Reference source not found. A). In the water maze space probe trial, the residence time of the ICV-STZ model rats in the target quadrant was significantly shortened. Compared with the model group, the residence time of the rats in the administration group with the intraperitoneal injection of edaravone (6 mg/kg) or edaravone and dexborneol (7.5 : 1, 5 : 1 and 2.5 : 1) compositions in the target quadrant was significantly prolonged, and the residence time in the edaravone and dexborneol (5 : 1 and 2.5 : 1) compositions was significantly superior to that in the edaravone group (Error! Reference source not found. B). Therefore, the edaravone and dexborneol (7.5 : 1, 5 : 1 and 2.5 : 1) compositions could significantly improve the deficits in the cognitive function of the ICV-STZ rats, and its efficacy was obviously superior to that of edaravone.

### Example 3: Effect of long-term administration of edaravone and dexborneol composition on AD cognitive function and AD pathology of 5 × FAD transgenic mice

### 1 Materials and methods

### 1.1 Experimental animals

Both wild-type (WT) mice and 5 × FAD transgenic mice were provided by Si majian research group of China Pharmaceutical University.

### 1.2 Test drugs and agents

Edaravone, i.e., edaravone injection ( 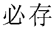 ^{®}, Simcere Pharmaceutical Co., Ltd.), active ingredient 3-methyl-1-phenyl-2-pyrazolin-5-one. Edaravone and dexborneol composition, i.e., edaravone and dexborneol concentrated solution for injection ( 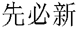 ^{®}, Simcere Pharmaceutical Co., Ltd.), with a mass ratio of 3-methyl-1-phenyl-2-pyrazolin-5-one to dexborneol being 4 : 1. A solvent control was 8% propylene glycol.

### 1.3 Experimental methods

In the experiment, the animals were divided into 4 groups. Administration was performed on the mice in each group from 8 weeks of age. After 16 weeks of continuous daily administration, water maze and Y maze behavioral tests were performed. After the behavioral tests were completed, the animals were sacrificed, and brain slice electrophysiology and AD pathology within the brain were tested, including the test of Aβ plaques, biomarkers such as GFAP and Iba-1, p-Tau, etc.

### 1.3.1 Animal grouping and administration

WT: solvent control group: 10 wild-type mice were given solvent control via i.p. at a dose of 10 mL/kg once daily.
5 × FAD: solvent group: ten 5 × FAD mice were given solvent control via i.p. at a dose of 10 mL/kg once daily.
5 × FAD: edaravone group: ten 5 × FAD mice were given edaravone ( 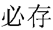 ^{®}) 6 mg/kg via i.p. at a dose of 10.0 mL/kg once daily.
5 × FAD: composition group: ten 5 × FAD mice were given 7.5 mg/kg edaravone and dexborneol concentrated solution for injection ( 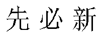 ^{®}) (edaravone : dexborneol = 4 : 1, containing 6 mg/kg edaravone and 1.5 mg/kg dexborneol) at a dose of 10.0 mL/kg once daily.

### 1.3.2 Water maze test:

Before the test, mice needed to be placed in the test room for about 30 min to acclimate the environment.

Experiment preparation: A constant temperature pool (120 cm in inner diameter and 50 cm in height) of a water maze was filled with clear water. The water temperature was controlled at about 18°C-22°C. The four apexes of the pool were selected and designated as "S", "N", "W" and "E", respectively. The platform was placed in the "SW" direction and 1-2 cm below the water surface. Titanium dioxide was added to the water to make the water opaque milky white.

Parameter setting: the test time was set to 1 min, and the residence time of mice on the platform was set to 2 s.

Training: The first 5 days were for the training phase of the water maze, the mice were placed in the pool in four different orientations every day, and their trajectories before boarding the platform, as well as the travel and time in each quadrant were recorded. For the mice that did not find the platform within 1 min, the test was completed after 1 min, they were guided to stay on the platform for 10-15 s. Specific mouse placement orientations were as shown in Table 1.

**Table 1 Summary of mouse placement orientations in water maze training test on different days**

| Days | Placement orientation | | | |
|---|---|---|---|---|
| 1 | N | E | SE | NW |
| 2 | SE | N | NW | E |
| 3 | NW | SE | E | N |
| 4 | E | NW | N | SE |
| 5 | N | SE | E | NW |

Testing: Testing was performed on the first day after the training was completed. During the testing, the platform was removed, the testing time was 60 s, the mouse was placed in the NE orientation, and the number of times the mouse crossed the platform and the time in the platform quadrant during the testing were recorded.

### 1.3.3 Y maze test:

Before the test, mice needed to be placed in the test room for about 30 min to acclimate the environment.

Training: One arm of the Y maze was blocked by a baffle, the mouse was placed at the end of one of the remaining two arms, allowing the mouse to probe freely for 15 min, and video recording was performed.

Testing: After 1 h of training, the baffle was removed, the channel of the Y maze was wiped with alcohol to remove the odor of the previous mouse, the mouse to be tested was placed in the same position for 5 min and video recording was performed, and the number of times the mouse entered the newly opened arm as a percentage of the total number of times it entered the three arms was recorded in the test.

### 1.3.4 Brain slice electrophysiology test

Electrophysiological recording was performed after the behavioral test of mice was completed. After anesthesia, all mice were euthanized, the brain tissues were rapidly collected and stored in 4°C oxygenated (95% O₂/5% CO₂) artificial cerebrospinal fluid (ACSF) (75 mM sucrose, 87 mM NaCl, 2.5 mM KCl), 1.25 mM NaH₂PO₄, 21.4 mM NaHCO₃, 0.5 mM CaCl₂, 7 mM MgCl₂ and 20 mM glucose. Hippocampal slices were cut by a vibrating knife in 4°C oxygenated ACSF, and in 32°C oxygenated ACSF. Next, the slices were incubated at room temperature for 1 hour before recording. Hippocampal LTP was induced by two theta burst stimulations (TBS). Data acquisition and analysis were performed using Clampex and Clampfit 8.2 (Molecular Devices).

### 1.3.5 AD pathology examination (immunohistochemical test)

After the behavioral test of animals was completed, the mice were subjected to cardiac perfusion followed by brain removal, and the brain was fixed in 4% paraformaldehyde for 24 h, dehydrated with 30% sucrose, snap-frozen and embedded with an embedding medium, and sliced (thickness 30 µM). The brain slices having the hippocampal region were selected, washed with PBS for 5 min × 3 times, permeabilized with 0.2% Triton, washed with PBS for 7 min × 3 times, and blocked with Blocking buffer (2.5% BSA, 0.3% Triton) for 1 h. The brain slices were placed in a primary antibody overnight at 4°C. On the second day, the brain slices in the primary antibody were taken out, washed with PBS for 5 min × 3 times, placed in a second antibody, and incubated for 1 h at room temperature in the dark. The brain slices were taken out and washed with PBS for 5 min × 3 times. An anti-fluorescence quencher containing DAPI was added dropwise, and the slices were mounted.

### 1.4 Statistical analysis

Data were expressed as Mean ± SEM. Statistical analysis between groups was performed using Prism software (GraphPad, USA): One-way or Two-way ANOVA was used for comparison of two or more groups, and then Fisher's LSD test was selected for analysis. P < 0.05 indicated significant difference. ##p < 0.01, ###p < 0.001, ####p < 0.0001, comparison with WT group; *p < 0.05, **p < 0.01, ***p < 0.0001, ****p < 0.0001, comparison with model group; & p < 0.05, &&p < 0.01, &&&&p < 0.0001, comparison between two groups indicated.

### 2. Experimental results

### 2.1 Improvement of the deficits in the cognitive function of AD transgenic mice by edaravone and dexborneol composition

In the Morris water maze test, compared with wild-type (WT) mice of the same age, 5 × FAD mice aged 24 weeks developed the deficits in the cognitive function, specifically indicated by: in the hidden platform training test, the escape latencies (from 2 to 5 days) of 5 × FAD mice were significantly longer than that of WT mice; in the platform withdrawal test, both the residence time in the target quadrant and the number of times mouse crossed the platform of 5 × FAD mice were significantly less than those of WT mice. Compared with the solvent control, 5 × FAD mice were given edaravone at 6 mg/kg or edaravone and dexborneol composition (containing 6 mg/kg edaravone and 1.5 mg/kg dexborneol) at 7.5 mg/kg via intraperitoneal injection once daily for 16 consecutive weeks from 8 weeks of age, which could significantly shorten the escape latency of the 5 × FAD mice in the water maze hidden platform test (Error! Reference source not found. A) and prolong the residence time in the target quadrant and the number of times the mouse crossed the platform in the platform withdrawal test (Error! Reference source not found. B,C). Compared with the edaravone administration group, the composition administration group had a shorter escape latency on day 5 of the hidden platform test, and a significantly longer residence time in the target quadrant and a greater number of times the mouse crossed the platform in the platform withdrawal test (Error! Reference source not found. A, B and C). This showed that both edaravone and the edaravone composition could improve the learning and memory cognitive function of the 5 × FAD mice in the water maze test, and the improvement effect of the composition was significantly superior to that of edaravone.

In the Y maze, compared with wild-type mice (WT) of the same age, 5 × FAD mice aged 24 weeks developed the deficits in the cognitive function, specifically indicated by: the number of times the mouse entered the newly opened arm of the 5 × FAD mice decreased significantly, that is, the spatial reference memory decreased. Compared with the solvent control, 5 × FAD mice were given edaravone at 6 mg/kg or edaravone and dexborneol composition (containing 6 mg/kg edaravone and 1.5 mg/kg dexborneol) at 7.5 mg/kg via intraperitoneal injection once daily for 16 consecutive weeks from 8 weeks of age, which could significantly increase the number of times the mouse entered the newly opened arm of the 5 × FAD mice. Compared with edaravone, the edaravone and dexborneol composition had a significantly higher efficacy in increasing the number of times the mouse entered the newly opened arm of the 5 × FAD mice (Error! Reference source not found.). This showed that both edaravone and the edaravone composition could improve the spatial reference memory cognitive function of the 5 × FAD mice in the Y maze test, and the improvement effect of the composition was significantly superior to that of edaravone.

### 2.2 Restoration of synaptic transmission function in hippocampus of AD mice by edaravone and dexborneol composition

long-term potentiation (LTP) reflects the plasticity of synaptic function and is one of the main mechanisms of learning and memory. After the water maze and Y maze tests were completed, the electrophysiological test showed that the hippocampal LTP in the 5 × FAD mice was significantly impaired. Compared with the 5 × FAD mouse group, the composition administration group could significantly restore the hippocampal LTP in mice, but the edaravone administration group had no obvious restoration effect on the hippocampal LTP in the 5 × FAD mice, and the LTP restoration in the composition administration group was also significantly superior to that of the edaravone group (Error! Reference source not found. A, B and C). This indicated that the synaptic plasticity in the hippocampus of the 5 × FAD transgenic mice aged 24 weeks was impaired, while the edaravone and dexborneol composition could promote hippocampal neuroplasticity and restore hippocampal LTP to a better level.

### 2.3 Improvement of AD pathology within the brain of AD mice by edaravone and dexborneol composition

The 5 × FAD mice aged 24 weeks developed obvious AD pathology (including β-amyloid plaques, astrogliosis, and microgliosis) within the brain, and tau protein hyperphosphorylation in hippocampal nerve cells. Compared with the solvent control group, 5 × FAD mice were given edaravone at 6 mg/kg or edaravone and dexborneol composition (containing 6 mg/kg edaravone and 1.5 mg/kg dexborneol) at 7.5 mg/kg via intraperitoneal injection once daily for 16 consecutive weeks from 8 weeks of age, which could significantly decrease AD pathology (β-amyloid plaques, astrogliosis, and microgliosis) within the brain and hippocampal tau protein hyperphosphorylation. Compared with the edaravone administration group, the edaravone and dexborneol composition had significant advantages in improving AD pathology (β-amyloid plaques, astrogliosis, and microgliosis) and reducing tau phosphorylation (Error! Reference source not found.).

## Claims

1. Use of a composition in improving or treating cognitive impairment, **characterized in that** the composition contains 3-methyl-1-phenyl-2-pyrazolin-5-one or a pharmaceutically acceptable salt thereof and borneol or dexborneol.

2. The use according to claim 1, **characterized in that** the composition contains 3-methyl-1-phenyl-2-pyrazolin-5-one or a pharmaceutically acceptable salt thereof and dexborneol.

3. The use according to claim 1, **characterized in that** the weight ratio of the 3-methyl-1-phenyl-2-pyrazolin-5-one or the pharmaceutically acceptable salt thereof to borneol or dexborneol is 10 : 1 to 1 : 8.

4. The use according to claim 1, **characterized in that** the weight ratio of the 3-methyl-1-phenyl-2-pyrazolin-5-one or the pharmaceutically acceptable salt thereof to borneol or dexborneol is 7.5 : 1 to 2.5 : 1.

5. The use according to claim 1, **characterized in that** the weight ratio of the 3-methyl-1-phenyl-2-pyrazolin-5-one or the pharmaceutically acceptable salt thereof to borneol or dexborneol is 5 : 1.

6. The use according to claim 1, **characterized in that** the weight ratio of the 3-methyl-1-phenyl-2-pyrazolin-5-one or the pharmaceutically acceptable salt thereof to borneol or dexborneol is 4 : 1.

7. The use according to claims 1 to 6, **characterized in that** the composition further comprises a pharmaceutically acceptable excipient.

8. The use according to claims 1 to 6, **characterized in that** the cognitive impairment refers to Alzheimer's disease, vascular dementia, mild cognitive impairment, and other types of dementia.

9. The use according to claims 1 to 6, **characterized in that** the cognitive impairment refers to Alzheimer's disease, vascular dementia, and mild cognitive impairment.

10. The use according to claims 1 to 6, **characterized in that** the cognitive impairment refers to Alzheimer's disease.
